(19) Europäisches Patentamt
European Patent Office
Office européen des brevets

(11) EP 1 944 063 A2

(12) **DEMANDE DE BREVET EUROPEEN**

(43) Date de publication:
**16.07.2008 Bulletin 2008/29**

(51) Int Cl.:
***A61Q 5/06*** *(2006.01)* ***A61K 8/87*** *(2006.01)*
***A61K 8/81*** *(2006.01)*

(21) Numéro de dépôt: **08300017.4**

(22) Date de dépôt: **09.01.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **12.01.2007 FR 0752655**

(71) Demandeur: **L'Oreal**
**75008 Paris (FR)**

(72) Inventeurs:
- **Benabddillah, Katarina**
**95130, LE PLESSIS-BOUCHARD (FR)**
- **Cothias, Pascale**
**78180, MONTIGNY-LE-BRETONNEUX (FR)**

(74) Mandataire: **Catherine, Alain et al**
**Cabinet Harlé & Phélip**
**7, rue de Madrid**
**75008 Paris (FR)**

# (54) Composition cosmétique comprenant un polymère cationique et un polymère anionique à chaîne hydrophobe

(57) La présente invention a pour objet une composition cosmétique comprenant, dans un milieu aqueux cosmétiquement acceptable :
(i) au moins un polyuréthane cationique comportant au moins un motif non ionique dérivé d'un homopolymère ou d'un copolymère d'oléfine, et
(ii) au moins un polymère anionique à chaîne hydrophobe.

L'invention a également pour objet l'utilisation d'une composition cosmétique telle que définie ci-dessus pour la formulation de lotions, de mousses, de crèmes, de laques ou de gels de coiffage, présentant notamment une très longue tenue dans le temps, une bonne élimination au shampooing, et une bonne résistance à l'humidité.

EP 1 944 063 A2

## Description

La présente invention concerne de nouvelles compositions cosmétiques, notamment de coiffage, comprenant l'association d'au moins un polyuréthane cationique comportant des motifs non ioniques dérivés d'au moins un homo- ou copolymère d'oléfine et au moins un polymère anionique à chaîne hydrophobe.

L'utilisation des polyuréthanes cationiques à caractère élastique dans des compositions cosmétiques, notamment des compositions de coiffage, est connue.

Ainsi la demande de brevet français FR 2 815 350 décrit des polyuréthanes cationiques à caractère élastique et leur utilisation pour la formulation de laques et de compositions de coiffage améliorant la souplesse de la coiffure, c'est-à-dire permettant d'obtenir une tenue des cheveux plus naturelle que celle obtenue avec des polymères fixants usuels.

La demande de brevet français FR 2 833 960 décrit des compositions cosmétiques de coiffage, et en particulier des compositions de coiffage à rincer, notamment des shampoings coiffants comportant un polyuréthane cationique ou amphotère auto-adhésif.

De manière surprenante, la Demanderesse a trouvé qu'en associant à ces polyuréthanes cationiques comportant des motifs dérivés d'un homo- et/ou copolymère d'oléfine, des polymères anioniques à chaîne hydrophobe, il était possible de formuler des compositions cosmétiques de coiffage conduisant à de bonnes propriétés cosmétiques et une élimination aisée au shampoing sans diminuer la fixation et la tenue dans le temps de la coiffure, avec une bonne résistance à l'humidité.

La demanderesse a également trouvé que de telles compositions cosmétiques assurent aux cheveux frisés, une diminution de volume lors d'un séchage libre de la coiffure.

L'invention a par conséquent pour objet une composition cosmétique, et en particulier une composition de coiffage, contenant, dans un milieu cosmétiquement acceptable, au moins un polyuréthane cationique comportant au moins un motif dérivé d'un homo- et/ou copolymère d'oléfine et au moins un polymère anionique à chaîne hydrophobe.

L'invention a également pour objet une telle composition contenant en outre un gaz propulseur et conditionné sous forme d'aérosol.

L'invention a encore pour objet un procédé de traitement des cheveux comprenant l'application sur les cheveux d'une première composition telle que définie ci-dessus, puis l'application d'une seconde composition, acide sur les cheveux, permettant de faire précipiter la première composition et augmenter ainsi la résistance de la coiffure à l'eau et au shampooing.

Selon l'invention, la composition cosmétique comprend, dans un milieu aqueux cosmétiquement acceptable :

(i) au moins un polyuréthane cationique comportant au moins une séquence non ionique dérivée d'un homopolymère ou d'un copolymère d'oléfine, et
(ii) au moins un polymère anionique à chaîne hydrophobe.

L'invention a également pour objet une telle composition contenant en outre un gaz propulseur et conditionné sous forme d'aérosol.

Le premier constituant essentiel des compositions de l'invention est le polyuréthane cationique comportant au moins un motif non ionique, dérivé d'un homo- ou co-polymère d'oléfine.

Les polyuréthanes cationiques préférés convenant pour l'invention comprennent :

(a) des motifs cationiques dérivés d'au moins un composé, de préférence une amine tertiaire ou quaternaire, présentant au moins deux fonctions réactives à hydrogène labile,
(b) des motifs non ioniques dérivés de polymères non ioniques partout à leurs extrémités des fonctions réactives à hydrogène labile, au moins un des motifs (b), de préférence au moins 50% en poids des motifs (b) et mieux la totalité des motifs (b) étant un motif ou des motifs (b1) dérivant d'un homo- ou copolymère d'oléfine portant à ses extrémités des fonctions réactives à hydrogène labile, et
(c) des motifs dérivés d'au moins un diisocyanate.

Par motif cationique, on entend au sens de la présente invention tout motif qui, soit par sa nature chimique propre, soit en fonction du milieu et/ou du pH dans lequel il se trouve, sera sous forme cationique.

On entend par fonctions réactives à hydrogène labile des fonctions capables, après départ d'un atome d'hydrogène, de former des liaisons covalentes avec les fonctions isocyanate des composés formant les motifs (b). On peut citer à titre d'exemple de telles fonctions les groupes hydroxyle, amine primaire ou amine secondaire ou encore les groupes thiol.

La polycondensation de composés portant ces fonctions réactives à hydrogène labile avec des composés comportant au moins deux fonctions isocyanate donne, selon la nature des fonctions réactives portant l'hydrogène labile

(-OH, $-NH_2$, -NHR ou -SH), respectivement des polyuréthanes, des polyurées ou des polythiouréthanes. Ainsi, les polymères utilisés dans l'invention peuvent être des copoly uréthane/urée et/ou thiouréthane.

Tous ces polymères sont regroupés dans la présente demande, par souci de simplification, sous le terme de polyuréthanes. De préférence, les polymères de l'invention sont de vrais polyuréthanes.

Lorsque la ou les amines tertiaires ou quaternaires formant les motifs (a) portent plus de deux fonctions à hydrogène labile, le polyuréthane obtenu présente une structure ramifiée.

Selon un mode de réalisation préféré, la ou les amines tertiaires ou quaternaires formant les motifs cationiques (a) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant une faible proportion d'amines portant plus de deux fonctions réactives à hydrogène labile.

La ou les amines tertiaires ou quaternaires formant les motifs cationiques (a) sont de préférence choisies parmi les composés correspondant à l'une des formules suivantes :

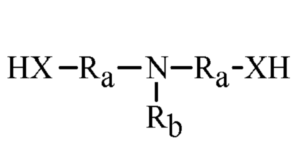
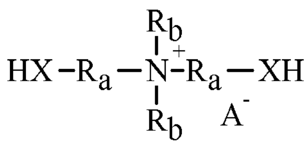
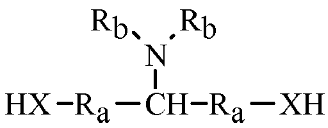
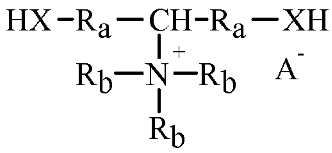
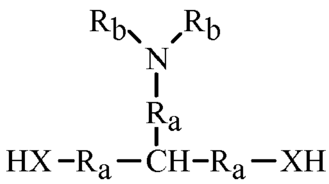
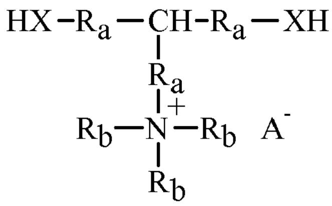

dans lesquelles

chaque $R_a$ représente indépendamment un groupe alkylène en $C_1$-$C_6$, linéaire ou ramifié, cycloalkylène en $C_3$-$C_6$ ou arylène, ou leurs mélanges ; tous pouvant être substitués par un ou plusieurs atomes d'halogène et comporter un ou plusieurs hétéroatomes choisis parmi O, N, P et S,

chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs hétéroatomes choisis parmi O, N, P et S,

chaque X représente indépendamment un atome d'oxygène ou de soufre ou un groupe NH ou $NR_c$, où $R_c$ représente un groupe alkyle en $C_1$-$C_6$, et

$A^-$ représente un contre-ion physiologiquement acceptable.

On peut citer à titre d'amines tertiaires particulièrement préférées pour l'obtention du polyuréthane cationique de l'invention défini ci-dessus, la N-méthyldiéthanolamine et la N-tert-butyldiéthanolamine.

La ou les amines tertiaires et quaternaires formant les motifs cationiques (a) du polyuréthane défini ci-dessus peuvent également être des polymères à fonction(s) amine tertiaire et/ou quaternaire, portant à leurs extrémités des fonctions réactives à hydrogène labile choisies parmi -OH, $-NH_2$, $-NHR_c$ ou -SH, où $R_c$ représente un groupe alkyle en $C_1$-$C_6$. La masse molaire moyenne en poids de ces polymères à fonctions amine tertiaire et/ou quaternaire est de

préférence comprise entre 400 et 10 000.

On peut citer à titre d'exemples de tels polymères à fonction(s) amine appropriés les polyesters issus de la polycondensation de la N-méthyldiéthanolamine et de l'acide adipique.

Lorsque les amines formant les motifs cationiques (a) sont des composés à fonction(s) amine tertiaire, une partie ou la totalité de ces fonctions amine doit être neutralisée par un agent de neutralisation approprié choisi parmi les acides organiques ou minéraux physiologiquement acceptables. On peut citer à titre d'exemple d'acides préférés l'acide chlorhydrique, l'acide acétique et l'acide tartrique.

Le deuxième type de motifs formant les polyuréthanes préférés de la présente invention sont des motifs macromoléculaires, appelés motifs (b), dérivés de polymères non ioniques portant à leurs extrémités des fonctions réactives à hydrogène labile et ayant de préférence une température de transition vitreuse (Tg), mesurée par analyse enthalpique différentielle, inférieure à 10°C.

Selon l'invention, au moins un de ces motifs (b1) dérive d'un homo-ou copolymère d'oléfine.

Les propriétés viscoélastiques des polyuréthanes sont particulièrement avantageuses lorsque les motifs (b) sont dérivés de polymères ayant une température de transition vitreuse inférieure à 0°C et mieux encore inférieure à -10°C.

Ces polymères ont de préférence une masse molaire moyenne en poids comprise entre 400 et 10 000 et plus particulièrement entre 1000 et 5000.

Les polymères non ioniques susceptibles de former les motifs non ioniques (b2) différents des motifs non-ioniques (b1) dérivant des homo- et copolymères d'oléfine, peuvent être choisis parmi les polyéthers, les polyesters, les polysiloxanes, les polycarbonates et les polymères fluorés.

De préférence, les polymères susceptibles de former les motifs non-ioniques (b) sont choisis uniquement parmi les homo- et copolymères d'oléfine.

Parmi les polymères d'oléfine terminés par des groupes réactifs à hydrogène labile convenant pour la présente invention on peut citer les homopolymères et les copolymères statistiques ou blocs d'éthylène, de propylène, 1-butylène, 2-butylène, isobutylène, 1,2-butadiène, 1,4-butadiène et d'isoprène.

Les homo- et copolymères de butadiène et d'isoprène peuvent être partiellement ou totalement hydrogénés.

Les polymères préférés sont les copolymères d'éthylène/butylène, les polybutadiènes et les polybutadiènes hydrogénés porteurs de groupes réactifs à hydrogène labile à leurs extrémités et en particulier de groupes hydroxyles. Encore plus préférentiellement ces polymères sont des 1,2 et/ou 1,4 polybutadiènes.

De tels polymères sont disponibles commercialement par exemple sous la dénomination KRATON® L, en particulier KRATON® L 2203 (polybutadiènediol hydrogéné) auprès de la société KRATON polymers, KRASOL LBH® et LBHP®, notamment KRASOL LBHP® 2000 (polybutadiène diol) auprès de la société SARTOMER et GI® 3000 (copolymère éthylène/butylène) auprès de la société NISSO CHEMICAL.

Comme expliqué précédemment, le polyuréthane selon l'invention est formé par la réaction des composés (a) avec au moins un composé (b) comportant au moins deux fonctions isocyanate.

Les diisocyanates formant les motifs (c) englobent les diisocyanates aliphatiques, alicycliques ou aromatiques.

Des diisocyanates préférés sont choisis parmi le méthylènediphényldiisocyanate, le méthylènecyclohexanediisocyanate, l'isophoronediisocyanate, le toluènediisocyanate, le natphtalènediisocyanate, le butanediisocyanate et l'hexyldiisocyanate. Ces diisocyanates peuvent bien entendu être utilisés seuls ou sous forme de mélange de deux ou plusieurs diisocyanates. Encore plus préférentiellement le diisocyanate est l'isophoronediisocyanate.

Comme indiqué ci-avant, les polyuréthanes cationiques de la présente invention peuvent contenir, en plus des motifs (a), (b1) et (c), une certaine fraction de motifs (b2) dérivés de composés, en général monomères, non ioniques contenant au moins deux fonctions à hydrogène labile et différents des composés conduisant aux motifs (b1).

Ces motifs (b2) sont dérivés en général de diols en $C_1$-$C_{12}$, par exemple de néopentylglycol, d'hexaéthylèneglycol, de 1,2-éthanediol, de 1,2-propanediol et de 1,3-propanediol ou d'aminoalcools, en $C_1$-$C_6$ par exemple l'aminoéthanol.

Les polyuréthanes cationiques de l'invention présentent de préférence un caractère élastique.

Dans un mode de réalisation particulier de l'invention, le polyuréthane cationique ne comprend pas de motifs en plus des motifs (a), (b) et (c). Un polyuréthane répondant à cette définition est le polyuréthane (A) décrit dans les exemples.

Dans un mode de réalisation alternatif, le polyuréthane cationique comprend des motifs en plus des motifs (a), (b) et (c). Un polyuréthane répondant à cette définition est le polyuréthane (B) décrit dans les exemples.

La viscosité du polyuréthane cationique selon l'invention, mesurée à 10% dans le tétrahydrofuranne, à 25°C, avec un viscosimètre Brookfield, module aiguille, est généralement comprise entre 1 et 1000 cps, de préférence entre 1 et 100 cps, mieux entre 2 et 80 cps. Le polyuréthane est caractérisé sous forme non neutralisée.

Le paramètre physique caractérisant les propriétés visco-élastiques des polyuréthanes cationiques décrits précédemment sont leur recouvrance en traction. Cette recouvrance est déterminée par essai de fluage en traction consistant à étirer rapidement une éprouvette jusqu'à un taux d'allongement prédéterminé, puis à relâcher la contrainte

et à mesurer la longueur de l'éprouvette.

L'essai de fluage utilisé pour la caractérisation du polyuréthane cationique à caractère élastique selon l'invention se déroule de la manière suivante :

On utilise, comme éprouvette, un film du polyuréthane ayant une épaisseur de 500 ± 50 mm, découpé en bandes de 80 mm x 15 mm. Ce film de copolymère est obtenu par séchage, à une température de 22 ± 2 °C et à une humidité relative de 50 ± 5 %, d'une solution ou dispersion à 3 % en poids dudit polyuréthane dans de l'eau et/ou de l'éthanol.

Chaque bande est fixée entre deux mors, distants de 50 ± 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire jusqu'à l'obtention d'une bande présentant 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\ \varepsilon_{max}) \times 100$$

Le polyuréthane cationique à caractère élastique défini ci-dessus a de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (b) et des polyuréthanes cationiques de la présente invention est mesurée par analyse enthalpique différentielle (DSC, *differential scanning calorimetry)* selon la norme ASTM D3418-97.

Les polyuréthanes cationiques à caractère élastique de la présente invention présentent de préférence au moins deux températures de transition vitreuse, dont une au moins est inférieure à 10°C, de préférence inférieure à 0°C et mieux encore inférieure à -10°C, et au moins une autre est supérieure ou égale à la température ambiante (20°C).

La recouvrance instantanée et par conséquent les propriétés viscoélastiques des polyuréthanes de la présente invention dépend des fractions des différents motifs monomères (a), (b1), (b2) et (c).

La fraction de motifs (a) doit être de préférence suffisante pour conférer aux polymères leur charge positive responsable de leur bonne affinité pour les substrats kératiniques. Les motifs (b) doivent de préférence représenter une fraction en poids suffisante pour que les polyuréthanes présentent de préférence au moins une température de transition vitreuse inférieure à 10 °C et ne forment pas des films cassants.

De manière préférée, les motifs (a) représentent de 0,1 à 90 %, de préférence de 1 à 30 %, mieux de 5 à 25% et idéalement de 5 à 10% en poids, les motifs (b1) de 10 à 99,9 %, de préférence de 20 à 99 % et mieux de 30 à 85% en poids et les motifs (b2) de 0 à 50 % en poids, de préférence de 0 à 30 % en poids par rapport au poids total des motifs du polyuréthane. De préférence encore les polyuréthanes de l'invention ne comportent pas de motif (b2).

De manière préférée les motifs (c) représentent de 1 à 60% mieux de 5 à 50% idéalement de 10 à 40% du poids total des motifs polyuréthane

Les motifs (c) sont de préférence présents en une quantité essentiellement stoechiométrique par rapport à la somme des motifs (a) et (b). L'homme du métier saura choisir un éventuel excès molaire de l'un ou de l'autre type de fonction pour ajuster la masse molaire à la valeur désirée.

La quantité de polyuréthane présente dans une composition cosmétique objet de l'invention, dépend bien entendu du type de composition et des propriétés recherchées et peut varier à l'intérieur d'une gamme très large, comprise généralement de préférence entre 0,01 et 40 % en poids, de préférence entre 0,05 et 20 %, idéalement entre 0,1 et 10% en poids, rapporté à la composition cosmétique finale.

Le deuxième constituant essentiel des compositions de l'invention est un polymère anionique à chaîne hydrophobe.

Par chaîne hydrophobe on entend au sens de la présente invention une chaîne hydrocarbonée linéaire ou ramifiée, saturée ou insaturée comportant au moins 8 atomes de carbone. De préférence, cette chaîne est une chaîne alkyle ou alkényle.

Le ou les polymères anioniques à chaîne hydrophobe généralement utilisés dans la présente invention sont des polymères comportant des groupes dérivés d'acides carboxylique, sulfonique ou phosphorique, et présentant une masse moléculaire en poids comprise entre 500 et 5 000 000, et comportant au moins une chaîne grasse en $C_8$-$C_{30}$., de préférence en $C_{10}$-$C_{24}$ et mieux en $C_{12}$ - $C_{18}$.

On peut citer :

- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci, et dont le motif éther d'allyle à chaîne grasse correspond au monomère de formule (I) suivante :

$$CH_2 = CR'CH_2OB_nR \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl ($C_{18}$).

Des polymères à chaîne hydrophobe de ce type sont décrits et préparés, selon un procédé de polymérisation en émulsion, dans le brevet EP-0 216 479.

Parmi ces polymères à chaîne hydrophobe, on préfère particulièrement selon l'invention les polymères formés à partir de 20 à 60 % en poids d'acide acrylique et / ou d'acide méthacrylique, de 5 à 60 % en poids de (méth)acrylates d'alkyles inférieurs, de 2 à 50 % en poids d'éther d'allyle à chaîne grasse de formule (I), et de 0 à 1 % en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80® et SALCARE SC90® qui sont des émulsions aqueuses à 30 % d'un terpolymère réticulé d'acide méthacrylique, d'acrylate d'éthyle et de steareth-10-allyl éther (40 / 50 / 10).

- (II) ceux comportant au moins un motif hydrophile de type acide carboxylique insaturé oléfinique, et au moins un motif hydrophobe de type ester d'alkyl ($C_{10}$-$C_{30}$) d'acide carboxylique insaturé.

  De préférence, ces polymères sont choisis parmi ceux dont le motif hydrophile de type acide carboxylique insaturé oléfinique correspond au monomère de formule (II) suivante :

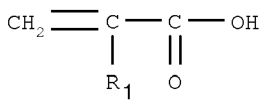

(II)

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl ($C_{10}$-$C_{30}$) d'acide carboxylique insaturé correspond au monomère de formule (III) suivante :

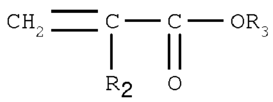

(III)

dans laquelle, $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_{10}$-$C_{30}$, et de préférence en $C_{12}$-$C_{22}$.

Des esters d'alkyles ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4509 949.

Parmi ce type de polymères à chaîne hydrophobe, on utilisera plus particulièrement des polymères formés à partir d'un mélange de monomères comprenant :

(i) essentiellement de l'acide acrylique,

(ii) un ester de formule (III) décrite ci-dessus et dans laquelle $R_2$ désigne H ou $CH_3$, $R_3$ désignant un radical alkyle ayant de 12 à 22 atomes de carbone,

(iii) et un agent réticulant, qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ce type de polymères à chaîne hydrophobe, on utilisera plus particulièrement ceux constitués de 95 à 60 % en poids d'acide acrylique (motif hydrophile), 4 à 40 % en poids d'acrylate d'alkyles en $C_{10}$-$C_{30}$ (motif hydrophobe), et 0 à 6 % en poids de monomère polymérisable réticulant, ou bien ceux constitués de 98 à 96 % en poids d'acide acrylique (motif hydrophile), 1 à 4 % en poids d'acrylate d'alkyles en $C_{10}$-$C_{30}$ (motif hydrophobe), et 0,1 à 0,6 % en poids de monomère polymérisable réticulant tel que ceux décrits précedemment.
Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

- (III) les terpolymères d'anhydride maléique / □-oléfine en $C_{30}$-$C_{38}$ / maléate d'alkyle tel que le produit (copolymère anhydride maléique / □-oléfine en $C_{30}$-$C_{38}$ / maléate d'isopropyle) vendu sous le nom PERFORMA V 1608® par la société NEWPHASE TECHNOLOGIES.
- (IV) les terpolymères acryliques comprenant :

(a) environ 20 % à 70 % en poids d'un acide carboxylique à insaturation □,□-monoéthylénique,

(b) environ 20 à 80 % en poids d'un monomère à insaturation □,□-monoéthylénique non-tensio-actif différent de (a),

(c) environ 0,5 à 60 % en poids d'un mono-uréthane non-ionique qui est le produit de réaction d'un tensio-actif monohydrique avec un monoisocyanate à insaturation monoéthylénique,

tels que ceux décrits dans la demande de brevet EP-A-0173109 et plus particulièrement celui décrit dans l'exemple 3, à savoir, un terpolymère acide méthacrylique / acrylate de méthyle / diméthyl métaisopropényl benzyl isocyanate d'alcool béhényle éthoxylé (40 OE) en dispersion aqueuse à 25 %.

- (V) les copolymères comportant parmi leurs monomères un acide carboxylique à insaturation □,□-monoéthylénique et un ester d'acide carboxylique à insaturation □,□-monoéthylénique et d'un alcool gras oxyalkyléné.
Préférentiellement ces composés comprennent également comme monomère un ester d'acide carboxylique à insaturation □,□-monoéthylénique et d'alcool en $C_1$-$C_4$.
A titre d'exemple de ce type de composé on peut citer l'ACULYN 22® vendu par la société ROHM et HAAS, qui est un terpolymère acide méthacrylique / acrylate d'éthyle / méthacrylate de stéaryle oxyalkyléné.
- (VI) les polymères comportant au moins un monomère à insaturation éthylénique à groupement sulfonique sous forme libre ou partiellement ou totalement neutralisé et comprenant au moins une partie hydrophobe. Ces polymères peuvent être réticulés ou non réticulés. De préférence, on choisit des polymères réticulés.
Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le diacrylate de tétraéthylèneglycol, la triallylamine, le méthylène-bis-acrylamide, le triméthylol propane triacrylate, le tétra-allyloxy-éthane, le triméthylolpropane-diallyléther, le méthacrylate d'allyle, les éthers allyliques d'alcools de la série des sucres, ou d'autres allyl- ou vinyl- éthers d'alcools polyfonctionnels, ou les mélanges de ces composés.
On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.
Les monomères à insaturation éthylènique à groupement sulfonique sont choisis notamment parmi l'acide vinylsulfonique, l'acide styrènesulfonique, les acides (méth)acrylamido($C_1$-$C_{22}$)alkylsulfoniques, les acides N-($C_1$-$C_{22}$)alkyl (méth)acrylamido($C_1$-$C_{22}$)alkylsulfoniques comme l'acide undécyl-acrylamido-méthane-sulfonique ainsi que leurs formes partiellement ou totalement neutralisées.
Plus préférentiellement, on utilisera les acides (méth)acrylamido($C_1$-$C_{22}$) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères (VI) conformes à l'invention peuvent notamment être choisis parmi les polymères amphiphiles statistiques d'AMPS modifiés par réaction avec une n-monoalkylamine ou une di-n-alkylamine en $C_6$-$C_{22}$, et tels que ceux décrits dans la demande de brevet WO00/31154 (faisant partie intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou polyalkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :

- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior- Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Les monomères hydrophobes à insaturation éthylénique de ces copolymères particuliers sont choisis de préférence parmi les acrylates ou les acrylamides de formule (IV) suivante :

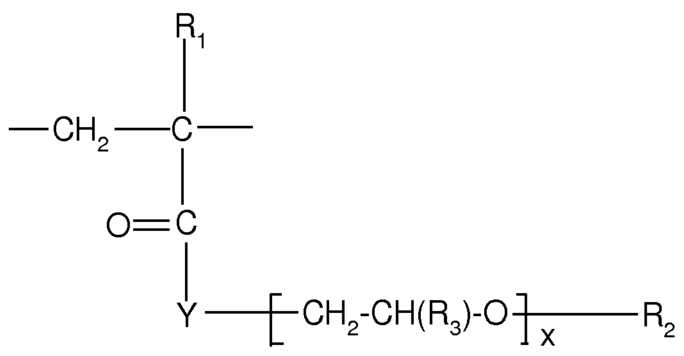

(I) (IV)

dans laquelle $R_1$ et $R_3$, identiques ou différents, désignent un atome d'hydrogène ou un radical alkyle linéaire ou ramifié en $C_1$-$C_6$ (de préférence méthyle) ; Y désigne O ou NH ; $R_2$ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical $R_2$ est choisi de préférence parmi les radicaux alkyles en $C_6$-$C_{18}$ linéaires (par exemple n-hexyle, n-octyle, n-décyle, n-hexadécyle, n-dodécyle), ramifiés ou cycliques (par exemple cyclododécane ($C_{12}$) ou adamantane ($C_{10}$)) ; les radicaux alkylperfluorés en $C_6$-$C_{18}$ (par exemple le groupement de formule $-(CH_2)_2-(CF_2)_9-CF_3$) ; le radical cholestéryle ($C_{27}$) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (IV) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 8 à 25.

Parmi ces polymères, on peut citer :

- les copolymères réticulés ou non réticulés, neutralisés ou non, comportant de 15 à 60% en poids de motifs AMPS et de 40 à 85% en poids de motifs ($C_8$-$C_{16}$)alkyl(méth)acrylamide ou de motifs ($C_8$-$C_{16}$)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-($C_6$-$C_{18}$)alkylacrylamide, tels que ceux décrits dans le brevet US-5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (V) suivante :

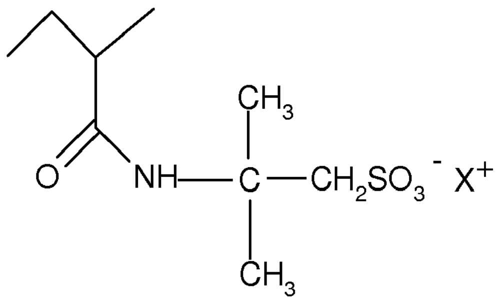

(V)

dans laquelle $X^+$ est un proton, un cation de métal alcalin, un cation alcalino-terreux ou l'ion ammonium, et de motifs de formule (VI) suivante :

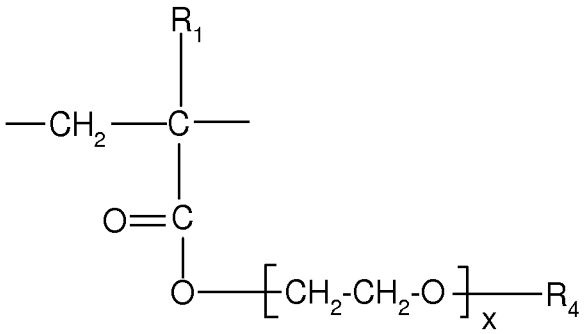

VI

dans laquelle x désigne un nombre entier variant de 3 à 100, de préférence de 3 à 50 et plus préférentiellement de 8 à 25 ; $R_1$ a la même signification que celle indiquée ci-dessus dans la formule (IV) et $R_4$ désigne un alkyle linéaire ou ramifié en $C_6$-$C_{22}$ et plus préférentiellement en $C_{12}$-$C_{18}$.

Les polymères particulièrement préférés sont ceux pour lesquels x = 25 , $R_1$ désigne méthyle et $R_4$ représente n-dodécyle ; ils sont décrits dans les articles de Morishima mentionnés ci-dessus.

La concentration molaire en % des motifs de formule (V) et des motifs de formule (VI) variera en fonction de l'application cosmétique souhaitée et des propriétés rhéologiques de la formulation recherchées. Elle varie de préférence de 70 à 99% en moles de motifs AMPS et de 1 à 30% en moles de motifs de formule (VI) par rapport au copolymère et plus particulièrement de 70 à 90% en moles de motifs AMPS et de 10 à 30% en moles de motifs de formule (VI).

Ces copolymères peuvent être obtenus par les procédés classiques de copolymérisation radicalaire en présence d'un ou plusieurs initiateurs tels que par exemple, l'azobisisobutyronitrile (AIBN), l'azobisdivaléronitrile, le ABAH

(2,2-azobis-[2-amidinopropane] hydrochloride) ou le peroxyde de dilauryle.

- (VII) Les copolymères comprenant à titre de monomère au moins un acide carboxylique à insaturation monoéthylénique, au moins un ester d'un tel acide avec un alcool aliphatique en C8 à C30 et au moins un vinyllactame. De préférence, le vinyllactame est la vinylpyrrolidone. A titre d'exemple d'un tel polymère, on peut citer l'ACRYLIDONE LM qui est un terpolymère acide acrylique / monoacrylate de lauryle / vinylppyrrolidone.

De préférence, le polymère anionique à chaîne grasse de l'invention est l'un de ceux de la famille (VII)
De préférence, le ou les polymères anioniques à chaîne hydrophobe représentent de 0,01 à 20% de préférence, de 0,1 à 5% du poids total de la composition.

Le pH de la composition appliquée sur les matières kératiniques est généralement compris entre les valeurs 2 et 11. Il est de préférence compris entre 3 et 8, et peut être ajusté à la valeur désirée au moyen d'agents acidifiants ou alcalinisants bien connus de l'état de la technique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante :

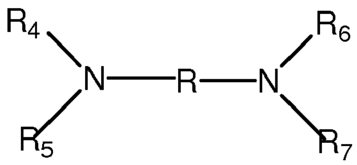

dans laquelle R est un reste propylène éventuellement substitué par un groupement hydroxyle ou un radical alkyle en C1-C4 ; R4, R5, R6 et R7, identiques ou différents, représentent un atome d'hydrogène, un radical alkyle en C1-C4 ou hydroxyalkyle en C1-C4. De préférence, une triéthanolamine est utilisée comme agent alcalinisant dans les compositions selon l'invention.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

Les compositions selon l'invention peuvent également contenir d'autres adjuvants cosmétiquement acceptables, tels que par exemple des agents tensioactifs, des agents épaississants, des agents de pénétration, des parfums, des tampons, et divers adjuvants usuels comme des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, des agents réducteurs, des émulsionnants, des conservateurs, des charges, des filtres solaires, des protéines, des polymères fixants anioniques, non ioniques, cationiques ou amphotères, des agents hydratants, des émollients, des agents adoucissants, des agents anti-mousse, des agents antiperspirants, des agents anti-radicaux libres, des polymères fixants ou non, des bactéricides, des séquestrants, des antipelliculaires, des anti-oxydants, des polyols, et tout autre additif classiquement utilisé dans les compositions cosmétiques destinées à être appliquées sur les cheveux.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de $5.10^{-6}$ à $2,5m^2/s$ à 25°C et de préférence $1.10^{-5}$ à $1\ m^2/s$.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

(i) les polydiolkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATINE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi

que leurs mélanges.

On peut également citer les cyclocopolymères du type diméthylsiloxanes/ méthylalkylsiloxane, tel que la "SILICONE VOLATILE FZ 3109" commercialisée par la société UNION CARBIDE, de formule :

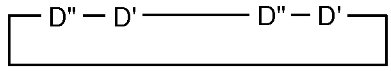

avec D'' : 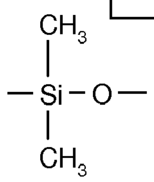 avec D' : 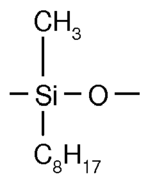

On peut également citer les mélanges de polydialkyl siloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les polydialkylsiloxanes volatiles linéaires ayant 2 à 9 atomes de silicium et présentant une viscosité inférieure ou égale à $5.10^{-6}m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 $mm^2/s$;
- les huiles VISCASIL® de GENERAL ELECTRIC et certaines huiles des séries SF (SF 96, SF 18) de GENERAL ELECTRIC.

  On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl ($C_1$-$C_{20}$) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :

- les mélanges formés à partir d'un polydiméthylsiloxane hydroxylé en bout de chaîne (ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthyl-siloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}m^2/s$.

Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs:

$R_2SiO_{2/2}$, $R_3SiO_{1/2}$, $RSiO_{3/2}$ et $SiO_{4/2}$

dans lesquelles R représente un alkyle possédant 1 à 16 atomes de carbone. Parmi ces produits, ceux particulièrement préférés sont ceux dans lesquels R désigne un groupe alkyle inférieur en $C_1$-$C_4$, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl méthylphénylsiloxanes, les polydiméthyl diphénylsiloxanes linéaires et/ou ramifiés de viscosité allant de $1.10^{-5}$ à $5.10^{-2}m^2/s$ à 25°C.

Parmi ces polyalkylarylsiloxanes on peut citer à titre d'exemple les produits commercialisés sous les dénominations suivantes :

- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl ($C_{12}$)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

Les silicones telles que décrites ci-dessus peuvent être utilisées seules ou en mélange, en une quantité comprise entre 0,01 et 20% en poids, de préférence entre 0,1 et 5% en poids.

Les agents tensioactifs utilisables dans la composition selon la présente invention peuvent être des tensioactifs anioniques, non ioniques, amphotères ou cationiques, ou des mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéridesulfates ; les alkylsulfonates, les alkylamidesulfonates, les alkylarylsulfonates, les $\alpha$-oléfinesulfonates, les paraffine-sulfonates ; les alkylsulfosuccinates, les alkyléthersulfosuccinates, les alkylamidesulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en $C_6$-$C_{24}$ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl ($C_6$-$C_{24}$)éther-carboxyliques polyoxyalkylénés, les acides alkyl($C_6$-$C_{24}$)aryl($C_6$-$C_{24}$)éther-carboxyliques polyoxyalkylénés, les acides alkyl($C_6$-$C_{24}$)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les alkyl($C_6$-$C_{24}$)sulfates, les alkyl($C_6$-$C_{24}$)éthersulfates, les alkyl($C_6$-$C_{24}$)éthercarboxylates et leurs mélanges, par exemple le laurylsulfate d'ammonium, le laurylsulfate de sodium, le laurylsulfate de magnésium, le lauryléthersulfate de sodium, le lauryléthersulfate d'ammonium et le lauryléthersulfate de magnésium.

La composition selon la présente invention peut comprendre les agents tensioactifs anioniques en une quantité comprise de préférence entre 0,5 et 60% en poids, mieux encore entre 5 et 20% en poids par rapport au poids total de la composition.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl(C1-C20)phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl($C_6$-$C_{24}$)polyglucosides, les dérivés de N-alkyl($C_6$-$C_{24}$)glucamine, les oxydes d'amines tels que les oxydes d'alkyl($C_{10}$-$C_{14}$)amines ou les oxydes de N-acyl($C_{10}$-$C_{14}$)aminopropylmorpholine; et leurs mélanges.

Parmi les tensioactifs non-ioniques cités ci-dessus, on utilise de préférence les alkyl($C_6$-$C_{24}$)polyglycosides, en particulier le décylpolyglucoside.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl($C_8$-$C_{20}$)bétaïnes, les sulfobétaïnes, les alkyl($C_8$-$C_{20}$)amidoalkyl($C_6$-$C_8$)-bétaïnes ou les alkyl($C_8$-$C_{20}$)amidoalkyl($C_6$-$C_8$)sulfobétaïnes ; et leurs mélanges.

Parmi les dérivés d'amines, on peut citer les produits commercialisés sous la dénomination MIRANOL®, tels que décrits dans les brevets US-2,528,378 et US-2,781,354 et classés dans le dictionnaire CTFA, 3ème édition, 1982, sous les dénominations Amphocarboxy-glycinate et Amphocarboxypropionate de structures respectives (1) et (2) :

$$R_2\text{ -CONHCH}_2\text{CH}_2\text{ -N+}(R_3)(R_4)(\text{CH}_2\text{COO-}) \qquad (1)$$

dans laquelle :

$R_2$ désigne représente un groupe alkyle dérivé d'un acide $R_2$-COOH présent dans l'huile de coprah hydrolysée, un groupe heptyle, nonyle ou undécyle,
$R_3$ représente un groupe bêta-hydroxyéthyle, et
$R_4$ représente un groupe carboxyméthyle ;
Et

$$R_2\text{ -CONHCH}_2\text{CH}_2\text{ -N(B)(C)} \qquad (2)$$

dans laquelle :

B représente $-CH_2CH_2OX'$,

C représente -$(CH_2)_z$ -Y', avec z = 1 ou 2,
X' représente le groupement -$CH_2CH_2$-COOH ou un atome d'hydrogène,
Y' représente -COOH ou le groupe -$CH_2$ - CHOH - $SO_3H$,
$R_2$ représente le groupe alkyle d'un acide $R_{2'}$-COOH présent dans l'huile de coprah ou dans l'huile de lin hydrolysée, un groupe alkyle, notamment en $C_{17}$ et sa forme iso, un groupe $C_{17}$ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple on peut citer le cocoamphodiacétate commercialisé sous la dénomination commerciale MIRANOL® C2M concentré par la société RHODIA.
Parmi les tensioactifs amphothères, on utilise de préférence les alkyl($C_8$-$C_{20}$)bétaïnes telles que la cocobétaïne, les alkyl($C_8$-$C_{20}$)amidoalkyl($C_6$-$C_8$)bétaïnes telles que la cocamidobétaïne, les alkylamphodiacétates comme le cocoamphodiacétate disodique, et leurs mélanges.
La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.
Les agents tensioactifs non ioniques, amphotères et cationiques décrits ci-dessus peuvent être utilisés seuls ou en mélanges et leur quantité est comprise entre 0,1 % et 30% en poids, de préférence entre 0,5% et 25% en poids et mieux encore entre 1% et 20% en poids par rapport au poids total de la composition.
Les agents gélifiants et/ou épaississants convenant pour les compositions de l'invention sont bien connus dans la technique et peuvent être choisis parmi les polymères et copolymères carboxy-vinyliques, les polymères et copolymères (alkyl)acryliques, les polymères et copolymères (alkyl)acrylamides, les poly(oxyalkylène)glycols, les esters de poly(oxyalkylène)glycols, les alginates, les biosaccharides, les polysaccharides tels que les dérivés de cellulose et d'amidon, les gommes naturelles telles que les gommes de xanthane, de guar, de caroube, les scléroglucanes, les dérivés de chitine et de chitosane, les carraghénanes, les argiles, et leur mélanges.
A titre d'exemple d'agents gélifiants, notamment en phase aqueuse, on peut citer le SEPIGEL® 305 commercialisé par la société SEPPIC, le FUCOGEL® 1000 PP commercialisé par la société SOLABIA, le SYNTHALEN® K commercialisé par la société 3VSA, le LUVISKOL® VA 64 P commercialisé par la société BASF, l'HOSTACERIN® AMPS commercialisé par la société CLARIANT, le PEMULEN® TR1 commercialisé par la société GOODRICH, le LUBRAGEL® MS commercialisé par la société GUARDIAN, le SATIAGEL® KSO commercialisé par DEGUSSA et le KELTROL® commercialisé par la société KELCO.
L'agent gélifiant représente en général de 0,1 à 15%, de préférence de 0,5 à 10% en poids de la composition.
Les compositions de l'invention peuvent encore comporter des corps gras tels que les huiles minérales, végétales, animales et synthétiques, les cires et les esters d'acides gras.
Les compositions de l'invention peuvent encore comporter au moins une huile choisie parmi les huiles d'origine minérale, végétale, animale, synthétique, en particulier une huile hydrocarbonée et/ou siliconée et/ou fluorée, et leurs mélanges.
Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam® ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou

encore le bromoperfluorooctyle vendu sous la dénomination "FORALKYL®" par la Société Atochem ; le nonafluorométhoxybutane et le nonafluoroéthoxyisobutane ; les dérivés de perfluoromorpholine, tels que la 4-trifluorométhyl perfluoromorpholine vendue sous la dénomination "PF 5052®" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras saturés ou insaturés sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les esters gras sont notamment les esters d'acides carboxyliques en particulier les esters mono, di, tri ou tétracarboxyliques.

Les dérivés d'acides gras peuvent être en particulier des ester de polyéthylène glycol, en particulier le stéarate de polyéthylène glycol.

Les esters d'acides carboxyliques sont notamment les esters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle ; le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Comme alcools gras on peut citer les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;

Les corps gras représentent en général de 0,4 à 50%; de préférence 1 à 30%, et encore plus préférentiellement de 2 à 20% en poids de la composition totale.

Le milieu aqueux cosmétiquement acceptable peut également contenir des électrolytes minéraux ou organiques encore appelés sels.

Les électrolytes utilisés sont de préférence des sels hydrosolubles minéraux tels que les sels de métaux alcalins, alcalino-terreux, ou d'aluminium, d'acide chlorhydrique, sulfurique ou nitrique, ou bien des sels d'acides organiques tels que les carbonates, lactates, citrates ou tartrates de métaux alcalins, ou alcalino terreux ou d'aluminium. Les électrolytes particulièrement préférés sont choisis parmi le sulfate de potassium, le sulfate de sodium, le sulfate de magnésium, le nitrate de calcium, le nitrate de magnésium, le chlorure de sodium, le chlorure de potassium, le carbonate de potassium, le carbonate de potassium, le carbonate de sodium, et le citrate de sodium.

Ces électrolytes sont présents de préférence dans des proportions allant de 0,1% à 30% en poids, en particulier de 1 à 10% en poids, rapporté au poids total de la composition.

Le milieu aqueux cosmétiquement acceptable de la composition peut, outre de l'eau, également contenir un ou plusieurs solvants organiques.

Le solvant organique est généralement choisi parmi les olésols $C_1$-$C_6$ de préférence les alcanols tels que l'éthanol, le propanol et l'isopropanol, les alcanediols tels que le propylène glycol et les pentanediols, l'alcool benzylique, les alcanes en $C_5$-$C_{10}$, l'acétone, la méthyléthylcétone, l'acétate de méthyle, l'acétate de butyle, l'acétate d'éthyle, le diméthoxyéthane, le diéthoxyéthane et leurs mélanges.

En général, le solvant organique est présent à raison de 0,5 à 80% et de préférence de 1 à 50% du poids total de la composition.

L'homme du métier saurait ajouter les additifs sans perturber les propriétés des compositions de l'invention.

Les compositions de l'invention sont de préférence des compositions de se présentant sous forme de gel de coiffage, de crème ou de lait.

Elles peuvent également être conditionnées sous forme d'aérosol.

Lorsque la composition selon l'invention est conditionnée dans un dispositif aérosol, elle comprend au moins un agent propulseur, qui peut être choisi parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise un mélange isobutane / propane / butane.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

La composition selon l'invention peut notamment être utilisée en application non rincée sur les cheveux.

L'invention a également pour objet un procédé de traitement des cheveux comprenant l'application d'une composition cosmétique selon l'invention, et un procédé de mise en forme des cheveux, comprenant l'application d'une composition cosmétique selon l'invention. Ces procédés peuvent inclure une étape de rinçage.

L'invention a aussi pour objet un procédé de traitement des cheveux permettant d'augmenter la résistance de la coiffure à l'eau et au shampooing ledit procédé comprenant :

- l'application sur les cheveux d'une composition selon l'invention, et
- l'application d'une composition acide afin de faire précipiter la composition sur les cheveux.

De préférence, la composition acide est choisie parmi des acides minéraux, de préférence l'acide chlorhydrique, l'acide orthophosphorique, des acides carboxyliques et de préférence l'acide tartrique, l'acide citrique, l'acide lactique ou des acides sulfoniques.

Les exemples suivants illustrent la présente invention.

**Exemples de formulation**

**Exemple 1 : Gel-crème de coiffage**

**Tableau 1**

| | |
|---|---|
| Polyuréthane cationique à séquence polyoléfine[1] | 6% m.a. |
| ACRYLIDONE LM[2] | 1% m.a. |
| Triéthanolamine | QSP pH 6,5 |
| Eau déminéralisée | QSP 100% |
| [1]Polyuréthane (A) en dispersion aqueuse formé à partir de 8,7% de N-méthyl diéthanolamine, de 23,4% d'isophorone diisocyanate, de 67,9% de KRASOL LBH2000 (polybutadiène à fonctions terminales hydroxyles) et neutralisé à 40% par HCL. [2]Terpolymère acide acrylique / vinylpyrrolidone / méthacrylate de lauryle commercialisé par la société ISP. | |

La composition présente une texture de « gel-crème », et assure une longue tenue dans le temps lorsqu'elle est appliquée sur les cheveux. La composition assure également une très bonne élimination au shampooing. En diminuant

le pH, il est possible de changer de texture pour obtenir des compositions moins visqueuses du type crème ou lait.

La résistance à l'humidité est très bonne.

La composition peut être mise en oeuvre dans un procédé de traitement des cheveux permettant d'augmenter la résistance de la coiffure à l'eau et au shampooing, ledit procédé comprenant :

- l'application de la composition ci-dessus sur les cheveux,
- l'application d'une composition acide afin de faire précipiter la composition sur les cheveux,

de sorte à augmenter la résistance de la coiffure à l'eau et au shampooing.

**Exemple 2 : Mousse de coiffage selon l'invention**

**Tableau 2**

| | |
|---|---|
| Polyuréthane cationique à séquence polyoléfine[1] | 3% m.a. |
| ACRYLIDONE LM[2] | 0,5% m.a. |
| Triéthanolamine | 0,2% |
| Eau déminéralisée | QSP 100% |
| Mélange Isobutane/propane/ butane | 5% m.a. |
| [1] Polyuréthane (A) de l'exemple 1<br>[2]Terpolymère acide acrylique / vinylpyrrolidone / méthacrylate de lauryle commercialisé par la société ISP. | |

Appliquée sur les cheveux, cette mousse de coiffage assure une longue tenue dans le temps des coiffures sous forme de « brushing ». La composition assure également aux cheveux frisés, une diminution de volume et une diminution du dessin de boucle durable, lors d'un séchage libre de la coiffure.

La résistance à l'humidité est très bonne.

Un résultat similaire est obtenu en utilisant un polyuréthane (B) en dispersion aqueuse formé à partir de 8,4% de poly(tetraméthylène oxyde), 8,6% de N-méthyl diéthanolamine, de 21,4% d'isophorone diisocyanate, de 61,6% de KRATON L2203 polybutadiène à fonctions terminales hydroxyles) et neutralisé à 40% par HCL.

**Revendications**

1. Composition cosmétique comprenant, dans un milieu aqueux cosmétiquement acceptable :

    (i) au moins un polyuréthane cationique comportant au moins un motif non ionique dérivé d'un homopolymère ou d'un copolymère d'oléfine,
    (ii) au moins un polymère anionique à chaîne hydrophobe.

2. Composition cosmétique selon la revendication 1, **caractérisée en ce que** 50% en poids ou plus des motifs non ioniques du polyuréthane dérivent d'un homopolymère ou copolymère d'oléfine.

3. Composition cosmétique selon la revendication 1, **caractérisée en ce que** la totalité des motifs non ioniques du polyuréthane dérivent d'un homopolymère ou copolymère d'oléfine.

4. Composition cosmétique selon l'une quelconque des revendication 1 à 3, **caractérisée en ce** les homopolymères et copolymères d'oléfines sont des homopolymères et copolymères portant à leurs extrémités des fonctions à hydrogène labile, et présentant des motifs choisis parmi les motifs d'éthylène, de propylène, de 1-butylène, de 2-butylène, d'isobutylène, de 1,2-butadiène, de 1,4-butadiène, d'isoprène et leurs mélanges.

5. Composition cosmétique selon la revendication 4 **caractérisée en ce que** les homopolymères et copolymères d'oléfines sont issus de 1,2 et/ou 1,4 butadiène éventuellement hydrogénés.

**6.** Composition cosmétique selon l'une quelconque des revendications précédentes **caractérisée en ce que** le polyuréthane cationique comprend:

(a) des motifs cationiques résultant de la réaction d'au moins une amine tertiaire ou quaternaire présentant au moins deux fonctions réactives à hydrogène labile,
(b) des motifs non ioniques dont au moins un motif (b1) résulte de la réaction d'au moins un polymère choisi parmi les homopolymères et les copolymères d'oléfines portant à leurs extrémités des fonctions réactives à hydrogène labile et ayant une température de transition vitreuse (Tg), mesurée par analyse enthalpique différentielle, inférieure à 10°C, et
(c) des motifs résultant de la réaction d'au moins un diisocyanate.

**7.** Composition cosmétique selon la revendication 6, **caractérisée en ce que** les motifs cationiques (a) résultent de la réaction d'au moins une amine tertiaire ou quaternaire présentant deux fonctions réactives à hydrogène labile.

**8.** Composition cosmétique selon la revendication 7, **caractérisée en ce que** l'amine est choisie parmi les amines de formules suivantes :

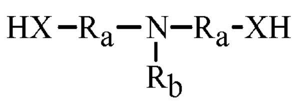

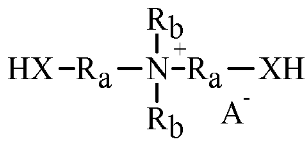

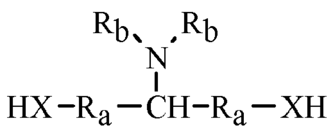

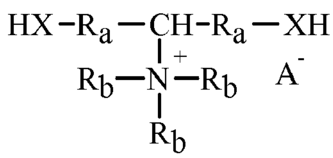

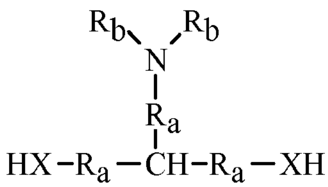

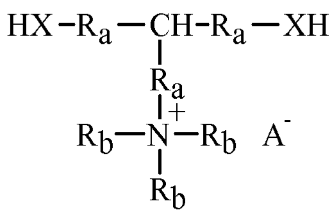

dans lesquelles
chaque $R_a$ représente indépendamment un groupe alkylène en $C_1$-$C_6$, linéaire ou ramifié, cycloalkylène en $C_3$-$C_6$ ou arylène, ou leurs mélanges ; tous pouvant être substitués par un ou plusieurs atomes d'halogène et comporter un ou plusieurs hétéroatomes choisis parmi O, N, P et S,
chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs atomes d'halogène et comporter un ou plusieurs hétéroatomes choisis parmi O, N, P et S,
chaque X représente indépendamment un atome d'oxygène ou de soufre ou un groupe NH ou $NR_c$, où $R_c$ représente un groupe alkyle en $C_1$-$C_6$, et
$A^-$ représente un contre-ion physiologiquement acceptable.

**9.** Composition cosmétique selon la revendication 8, **caractérisée en ce que** les motifs cationiques (a) résultent de la réaction de la N-méthyldiéthanolamine ou de la N-tert-butyldiéthanolamine.

**10.** Composition cosmétique selon la revendication 7, **caractérisée en ce que** les motifs (a) résultent de la réaction d'au moins un polymère à fonction(s) amine tertiaire et/ou quaternaire, portant à ses extrémités des fonctions réactives à hydrogène labile choisies parmi -OH, $-NH_2$, $-NHR_c$ ou -SH, et ayant une masse moléculaire en poids comprise entre 400 et 10 000, $R_c$ représentant un groupe alkyle en $C_1$-$C_6$.

**11.** Composition cosmétique selon l'une quelconque des revendications 6 à 10, **caractérisée en ce que** le polyuréthane cationique contient éventuellement au moins un motif non ionique (b2), différent du motif (b1), dérivant d'un composé non ionique monomère contenant au moins deux fonctions à hydrogène labile capables de réagir avec le ou lesdits composés (c) contenant au moins un diisocyanate.

**12.** Composition cosmétique selon la revendication 11, **caractérisée en ce que** les motifs cationiques (a) représentent de 0,1 à 90%, de préférence de 1 à 30%, mieux de 1 à 20% en poids, les motifs non-ioniques dérivés d'un homo- ou copolymère d'oléfine (b1) représentent de 10 à 99,9%, de préférence de 20 à 99% et mieux de 30 à 85% en poids, et les motifs non-ioniques (b2) représentent de 0 à 50%, de préférence 0 à 30% en poids du total des motifs du polyuréthane cationique.

**13.** Composition cosmétique selon l'une quelconque des revendications 6 à 11, **caractérisée en ce que** le diisocyanate est choisi parmi le méthylènediphényldiisocyanate, le méthylènecyclohexanediisocyanate, l'isophoronediisocyanate, le toluènediisocyanate, le naphtalènediisocyanate, le 1,4-butanediisocyanate et le 1,6-hexanediisocyanate.

**14.** Composition cosmétique selon la revendication 13, **caractérisée en ce que** le diisocyanate est l'isophoronediisocyanate.

**15.** Composition cosmétique selon l'une quelconque des revendications 6 à 14, **caractérisée en ce que** les motifs (c) représentent de 1 à 60%, de préférence de 5 à 50%, et idéalement 1 à 40% du poids total des motifs du polyuréthane cationique.

**16.** Composition cosmétique selon l'une quelconque des revendications 6 à 15, **caractérisée en ce que** le ou les composés non ioniques monomères formant les motifs non ioniques (b2) sont choisis parmi les diols en $C_1$-$C_{12}$, de préférence le néopentylglycol, l'hexa(éthylèneglycol), le 1,2-éthanediol, le 1,2-propanediol et le 1,3-propanediol, et les aminoalcools en $C_1$-$C_6$, de préférence l'aminoéthanol.

**17.** Composition cosmétique selon l'une quelconque des revendications 6 à 16, **caractérisée en ce que** le polyuréthane cationique ne comprend pas de motifs en plus des motifs (a), (b) et (c).

**18.** Composition cosmétique selon l'une quelconque des revendications 6 à 17, **caractérisée en ce que** le polyuréthane cationique présente un caractère élastique.

**19.** Composition selon l'une quelconque des revendications précédentes, **caractérisée en ce que** le (ou les) polyuréthane(s) cationique(s) représente(nt) de 0,01 % à 40%, de préférence 0,05 à 20% et idéalement de 0,1 à 10% en poids rapporté à la composition cosmétique finale.

**20.** Composition selon l'une quelconque des revendications précédentes, **caractérisée en ce que** le polymère anionique à chaîne hydrophobe est choisi parmi les polymères comportant des groupes dérivés d'acides carboxylique, sulfonique ou phosphorique, et présentant une masse moléculaire en poids comprise entre 500 et 5 000 000, et comportant au moins une chaîne grasse en $C_8$-$C_{30}$.

**21.** Composition selon l'une quelconque des revendications précédentes, **caractérisée en ce que** le polymère anionique à chaîne hydrophobe comporte au moins un monomère à insaturation éthylénique à groupement sulfonique, sous forme libre ou partiellement ou totalement neutralisée et en outre au moins une partie hydrophobe.

**22.** Composition selon la revendication 21, **caractérisée en ce que** le polymère anionique à chaîne hydrophobe comporte au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci, et dont le motif éther d'allyle à chaîne grasse correspond au monomère de formule (I) suivante :

$CH_2=CR'CH_2OB_nR$ (I)

dans laquelle R' désigne H ou $CH_3$, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (1) plus particulièrement préféré est un motif dans lequel R' désigne H, n est égal à 10, et R désigne un radical stéaryl ($C_{18}$).

**23.** Composition selon la revendication 21, **caractérisée en ce que** le polymère anionique à chaîne hydrophobe comporte au moins un motif hydrophile de type acide carboxylique insaturé oléfinique, et au moins un motif hydrophobe de type ester d'alkyl ($C_{10}$-$C_{30}$) d'acide carboxylique insaturé.

**24.** Composition selon la revendication 21, **caractérisée en ce que** le polymère anionique à chaîne hydrophobe et choisi parmi les terpolymères d'anhydride maléique/α-oléfine en $C_{30}$-$C_{38}$/maléate d'alkyle.25. Composition selon la revendication 21, **caractérisée en ce que** le polymère anionique à chaîne hydrophobe et choisi parmi les terpolymères acryliques comprenant :

(a) environ 20 % à 70 % en poids d'un acide carboxylique à insaturation α,β-monoéthylénique,
(b) environ 20 à 80 % en poids d'un monomère à insaturation α,β-monoéthylénique non-tensio-actif différent de (a),
(c) environ 0,5 à 60 % en poids d'un mono-uréthane non-ionique qui est le produit de réaction d'un tensio-actif monohydrique avec un monoisocyanate à insaturation monoéthylénique.

**25.** Composition selon la revendication 21, **caractérisée en ce que** le polymère anionique à chaîne hydrophobe et choisi parmi les copolymères comportant parmi leurs monomères un acide carboxylique à insaturation α,β-monoéthylénique et un ester d'acide carboxylique à insaturation α,β-monoéthylénique et d'un alcool gras oxyalkyléné.

**26.** Composition selon la revendication 21, **caractérisée en ce que** le polymère anionique à chaîne hydrophobe et choisi parmi les polymères comportant au moins un monomère à insaturation éthylénique à groupement sulfonique sous forme libre ou partiellement ou totalement neutralisé et comprenant au moins une partie hydrophobe. Ces polymères peuvent être réticulés ou non réticulés. De préférence, on choisit des polymères réticulés.

**27.** Composition selon la revendication 21, **caractérisée en ce que** le polymère anionique à chaîne hydrophobe et choisi parmi Les copolymères comprenant à titre de monomère au moins un acide carboxylique à insaturation monoéthylénique, au moins un ester d'un tel acide avec un alcool aliphatique en C8 à C30 et au moins un vinyllactame.

**28.** Composition selon l'une quelconque des revendications 21 à 28, **caractérisée en ce que** le ou les polymères anioniques à chaîne hydrophobe représentent de 0,01 à 20% de préférence, de 0,1 à 5% du poids total de la composition.

**29.** Composition selon l'une quelconque des revendications précédentes **caractérisée en ce qu'**elle comprend un ou plusieurs additifs choisis parmi les agents alcalinisant, les agents tensioactifs, les silicones, les sels, les solvants organiques, les parfums, les huiles minérales, végétales et/ou synthétiques, les esters d'acide gras, les agents de stabilisation de pH, les agents conservateurs et les agents absorbants les UV

**30.** Composition selon l'une quelconque des revendications précédentes **caractérisée en ce qu'**elle contient un gaz propulseur et est conditionnée sous forme d'un aérosol.

**31.** Procédé de coiffage ou de traitement des cheveux comprenant l'application d'une composition selon l'une quelconque des revendications 1 à 30 sur les cheveux, le rinçage éventuel des cheveux, puis la mise en forme et le séchage des cheveux.

**32.** Procédé de traitement des cheveux permettant d'augmenter la résistance de la coiffure à l'eau et au shampooing, ledit procédé comprenant :

- l'application d'une composition selon l'une quelconque des revendications 1 à 30, et
- l'application d'une composition acide afin de faire précipiter la composition sur les cheveux.

**33.** Procédé de traitement des cheveux selon la revendication 33, **caractérisée en ce que** la composition acide est choisie parmi des acides minéraux, de préférence l'acide chlorhydrique, l'acide orthophosphorique, des acides carboxyliques et de préférence l'acide tartrique, l'acide citrique, l'acide lactique ou des acides sulfoniques.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2815350
- FR 2833960
- EP 0216479 A
- US 3915921 A
- US 4509949 A
- EP 0173109 A
- WO 0031154 A
- EP 750899 A
- US 5089578 A
- US 2528378 A
- US 2781354 A
- JP 2295912 A


### Littérature non-brevet citée dans la description


- Self-assembling amphiphilic polyelectrolytes and their nanostructures - Chinese. *Journal of Polymer Science,* 2000, vol. 18 (40), 323-336
- Miscelle formation of random copolymers of sodium 2-(acrylamido)-2-methylpropanesulfonate and a non-ionic surfactant macromonomer in water as studied by fluorescence and dynamic light scattering. *Macromolecules,* 2000, vol. 33 (10), 3694-3704
- Solution properties of miscelle networks formed by non-ionic moieties covalently bound to an polyelectrolyte : salt effects on rheological behavior. *Langmuir,* 2000, vol. 16 (12), 5324-5332
- Stimuli responsive amphiphilic copolymers of sodium 2-(acrylamido)-2-methylpropanesulfonate and associative macromonomers. *Polym. Preprint, Div. Polym. Chem.,* 1999, vol. 40 (2), 220-221
- **WALTER NOLL.** Chemistry and Technology of Silicones. Academie Press, 1968
- **TODD ; BYERS.** Volatile Silicone fluids for cosmetics. *Cosmetics and Toiletries,* Janvier 1976, vol. 91, 27-32
- **M.R. PORTER.** Handbook of Surfactants. Blackie & Son, 1991, 116-178
- dictionnaire CTFA. 1982